# FASCICULE DE BREVET EUROPEEN

(11) **EP 1 194 044 B1**
(45) Date de publication et mention de la délivrance du brevet: **12.01.2005**
(21) Numéro de dépôt: 01911809.0
(22) Date de dépôt: 28.02.2001
(51) Int. Cl.: A23L 1/28

(54) **COMPOSITIONS PHARMACEUTIQUES OU DIETETIQUES A BASE DE CHAMPIGNONS ET LEUR UTILISATION**
PHARMAZEUTISCHE ODER DIÄTETISCHE ZUSAMMENSETZUNGEN AUS PILZEN UND DEREN VERWENDUNG
PHARMACEUTICAL OR DIETETIC MUSHROOM-BASED COMPOSITIONS AND THEIR USE

(30) Priorité: 28.02.2000 FR 0002460
(43) Date de publication de la demande: 10.04.2002
(73) Titulaire: Medecine Information Formation S.A., 51350 Cormontreuil (FR)
(72) Inventeur: DONATINI, Bruno, F-51350 Cormontreuil (FR)
(74) Mandataire: Burtin, Jean-François
(86) Numéro de dépôt international: PCT/FR2001/000577
(87) Numéro de publication internationale: WO 2001/064057

(56) Documents cités:
- DE-A- 4 141 889
- PATENT ABSTRACTS OF JAPAN vol. 1997, no. 04, 30 avril 1997 (1997-04-30) & JP 08 322506 A (GAIA:KK), 10 décembre 1996 (1996-12-10)
- DATABASE WPI Section Ch, Week 199631 Derwent Publications Ltd., London, GB; Class B04, AN 1996-303803 XP002152640 & JP 08 131120 A (TANAKA H), 28 mai 1996 (1996-05-28)
- DATABASE WPI Section Ch, Week 199901 Derwent Publications Ltd., London, GB; Class D13, AN 1999-002784 XP002152641 & JP 10 276718 A (MAEDA T), 20 octobre 1998 (1998-10-20)
- PATENT ABSTRACTS OF JAPAN vol. 1997, no. 10, 31 octobre 1997 (1997-10-31) & JP 09 149774 A (MICRO ARUJIE CORP KK), 10 juin 1997 (1997-06-10)
- DATABASE WPI Section Ch, Week 200045 Derwent Publications Ltd., London, GB; Class B04, AN 2000-500998 XP002152642 & JP 2000 169386 A (SARUDE YAKUHIN KK), 20 juin 2000 (2000-06-20)
- 'RÖMPP LEXIKON LEBENSMITTELCHEMIE', 1995, GEORG THIEME VERLAG, STUTTGART page 177

## Description

La présente invention se rapporte au domaine de la pharmacie et de la diététique.

La présente invention a plus particulièrement pour objet de nouvelles compositions pharmaceutiques et/ou diététiques à base de champignons.

Elle a spécifiquement pour objet des compositions pharmaceutiques et/ou diététiques associant un ou plusieurs champignons comestibles présentant des vertus thérapeutiques et du chitosane.

Avec le développement, en particulier dans les sociétés occidentales, de certaines maladies liées aux problèmes d'environnent ou à une mauvaise alimentation comme le cancer, le diabète ou les maladies cardio-vasculaires, une partie du corps médical rejoint en cela les nutritionnistes, et insiste de plus en plus sur l'importance d'un régime alimentaire sain pour prévenir, et même pour lutter contre ces maladies.

Parmi les divers aliments mis en avant, les champignons comestibles présentent des propriétés thérapeutiques intéressantes. Ainsi, ils posséderaient, suivant les cas, des propriétés immunostimulantes, amaigrissantes, hypoglycémiantes, antihypertensives, même anticancéreuses et antivirales. De plus en plus de travaux scientifiques viennent corroborer l'intérêt médical de ceux-ci.

Les champignons contiennent en moyenne :
- de 0,5 à 1,5 % de substances minérales telles que le potassium, le calcium, le magnésium, le phosphore, le zinc, le cuivre, le soufre, le sodium et surtout le sélénium;
- environ 4 % de protéines assimilables, notamment de la lysine ;
- environ 3,5 % de glucides tels que des polysaccharides, des lectines contenant des quantités importantes de galactose, de xylose, d'arabinose, de fucose, de rhamnose et de mannose ;
- de 0,05 à 2 % de lipides ;
- des vitamines et en particulier des vitamines du groupe B, à savoir la vitamine B1, la vitamine B2, l'acide pantothénique (vitamine B5), l'acide folique (vitamine B9), l'acide nicotinique ( vitamine B3 ) ; de la vitamine C ; de la vitamine D2 ; de la vitamine E ; de la vitamine PP et de la vitamine K.

Les principes actifs des champignons sont des polysaccharides ramifiés complexes ou des protéines fortement glycosylées, neutres ou faiblement basiques.

Cependant, les champignons présentent l'inconvénient majeur d'accumuler les substances toxiques de toute sorte. En effet, ils concentrent les minéraux et notamment les métaux lourds, en particulier le plomb, le cadmium, le manganèse, l'arsenic, le mercure.

Or la consommation de ces métaux lourds n'est pas sans incidence sur la santé. Ainsi :
- l'intoxication au plomb est notamment à l'origine du saturnisme soit sous sa forme aiguë qui se traduit par de violentes douleurs intestinales soit sous sa forme chronique qui se manifeste par des troubles nerveux, des néphrites interstitielles, des troubles hématologiques, la cachexie saturnine et qui peut conduire à la mort ;
- l'excès de manganèse peut provoquer la maladie de Parkinson comme le rapportent Huang CC et al. (Neurology 1998 ;50 :698-700) ;
- la toxicité du Cadmium est importante et peut induire des vomissements, des douleurs abdominales, des diarrhées sévères et des chutes de tension. A la suite d'un contact prolongé, il peut se fixer sur les groupes thiol des protéines, dans les hématies, dans le rein, en provoquant de la tubulopathie proximale, mais également dans les os et le foie. Il serait ainsi à l'origine de cancers comme celui du foie et de la prostate ;
- l'arsenic qui est contenu dans des insecticides et les fongicides peut provoquer des vomissements, des douleurs abdominales, des cyanoses, des difficultés respiratoires...
- le mercure, par exemple le méthyl-mercure, déclenche des vomissements sanglants, des spasmes abdominaux, des diarrhées sanglantes et des insuffisances rénales. Un sujet exposé de manière chronique au mercure peut présenter des troubles digestifs, rénaux, cutanés ou nerveux. Une intoxication aiguë au mercure peut se traduire par une néphropathie tubulo-interstitielle, une encéphalopathie ou une anurie.

Les champignons concentrent également les désherbants, les engrais et les insecticides.

Enfin, les champignons présentent également des taux de radioactivité plus ou moins importants suivant leur nature, leur équipement enzymatique, l'implantation de leur habitat (prairie, clairière, forêt de feuillus ou de conifères) sur le sol ou sur les souches ou encore suivant la profondeur d'implantation du mycélium.
En effet, cette radioactivité procède essentiellement de la faculté qu'ont les champignons, contrairement aux végétaux chlorophylliens, de concentrer également les métaux radioactifs, comme le Césium 134, le Césium 137, le Plomb 210 et le Radium 226.

Or, la consommation de tels métaux radioactifs - par exemple le césium 137 dont la demi-vie est de près de 30 ans - au-delà des normes européennes augmenterait le risque de cancer, le nombre de malformations congénitales ou d'anomalies génétiques transmissibles de génération en génération (fiche CRII-RAD (Commission de Recherche et d'Information Indépendantes sur la Radioactivité) n°3 novembre 1997).

Ainsi si on veut en faire une utilisation pharmaceutique ou même alimentaire en toute sécurité, il est actuellement nécessaire de réaliser l'extraction des principes actifs des champignons afin de prévenir de possibles contaminations ou de s'assurer de l'absence de ces dernières en réalisant des analyses telles que par exemple une analyse par spectrométrie gamma après dessiccation.
Or, aussi bien les opérations d'extraction que d'analyse sont longues, fastidieuses et par voie de conséquence d'un coût élevé.

Le problème de la présente invention est de réaliser des compositions à base de champignons qui n'ont pas à subir de traitements préalables pour les débarrasser d'éventuelles contaminations provoquées par des métaux lourds, des métaux radioactifs des désherbants, des engrais et des insecticides, ou qui n'ont pas été préalablement choisis spécialement pour s'assurer de l'absence d'agents contaminants.

L'invention consiste donc à associer dans des compositions pharmaceutiques ou diététiques, un ou plusieurs champignons ou parties de champignons comestibles, présentant des vertus thérapeutiques (vitamines, composés, manifestant des propriétés diététiques ou thérapeutiques) et du chitosane, comme défini à la revendication 1 sans avoir besoin de réaliser un traitement préalable des champignons du type purification comme par exemple une extraction ou une purification sur colonne.

Le demandeur a en effet constaté que le chitosane acide et/ou alcalin permet une libération progressive des principes actifs contenus dans le ou les champignons et assure une chélation des agents contaminants.

En outre, la libération des principes actifs du champignon est progressive et se traduit ainsi par une action et une efficacité qui se prolongent dans le temps en comparaison de l'effet obtenu par la prise d'un extrait isolé du même champignon.

Le chitosane présente une structure chimique proche de celle de la cellulose et comme les fibres végétales, il n'est pas digéré par l'organisme humain. C'est un composant naturel et il permet d'accroître la teneur des champignons en fibres. Le chitosan se fixe sur le squelette pré-existant du champignon et sur les polysaccharides en fonction du pH. Il sera relargué dans le tube digestif également en fonction des pH qui y règnent.

La supplémentation des champignons cultivés, en chitosane, peut être modérée ou importante, et dépasser éventuellement la teneur naturelle en chitine et en chitosane des champignons sauvages souvent plus ligneux et donc plus riches en éléments structurels de soutien.

Le chitosane est un polysaccharide linéaire constitué d'une longue chaîne de glucosamines liées par des ponts β(1-4)glucosidiques. Il a la structure chimique suivante :

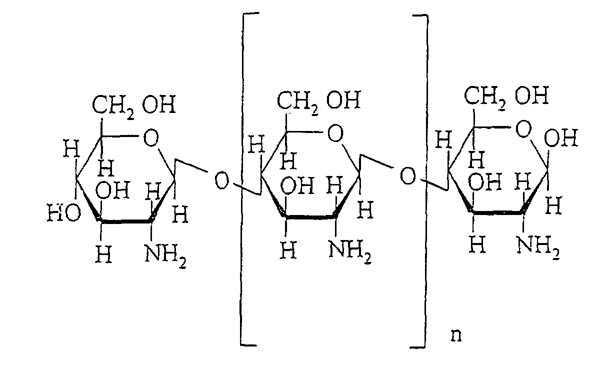

Il résulte de la chitine par désacétylation. Or, la chitine est le second plus important polysaccharide présent dans la nature après la cellulose et on la trouve aussi bien dans l'exosquelette de crustacés, de myriapodes et d'arthropodes que chez les insectes et dans les champignons.

Le taux de désacétylation peut varier de 80 à 100 % suivant la variété de chitosane et son poids moléculaire moyen est compris entre 5 000 et 1 000000 permettant d'assurer la solubilité, la turbidité et la viscosité des compositions conformes à l'invention.

Outre le fait d'être non-toxique et non-allergisant, il présente en plus l'avantage d'être antibactérien, antifongique et antiviral. L'utilisation du chitosane dans le domaine pharmaceutique a déjà été rapportée dans de nombreuses publications.

Ainsi, on a observé que le chitosane acide ou alcalin permettrait à la fois de diminuer le LDL cholestérol qui, en excès, peut se déposer dans les tissus, en particulier sur les parois des artères et d'augmenter le HDL cholestérol qui est considéré comme le «bon» cholestérol (Maezaki Y, Tsuji K, Nakagawa Y, et al. B Bioscience Biotechnology Biochem 1993; 57(9):1439-44).

D'autres auteurs ont confirmé que le chitosane apparaît comme étant un agent hypocholestérolémiant efficace, en étudiant son action chez les rats (Sugano et al Nutritional Rep. Int., 1978 ;18(5) :531-7).
Une publication chinoise signale que le chitosane pourrait également avoir des effets importants sur le métabolisme du glucose chez le rat.

D'autres auteurs encore fondent des espoirs importants sur le chitosane dans la lutte contre le SIDA. En effet, ces études ont montré que ce composé ralentit la synthèse des protéines du virus du SIDA dans des cultures de cellules humaines et de souris (Gama Sosa et al. Biochemical and Biophysical Research Communications ; 174 : 486-489).

JP-A-08 322 506 décrit déjà une préparation alimentaire formée de chitosane et du champignon Grifola frondosa. Cette préparation est décrite comme ayant d'excellentes propriétés immunologiques.

Le chitosane selon l'invention est utilisé sous forme acide ou cationique, c'est-à-dire présentant un pH inférieur à 6. Pour ceci, on ajoute à la chitine un acide organique choisi notamment parmi l'acide acétique, l'acide lactique, l'acide succinique, l'acide tartrique, l'acide ascorbique, l'acide citrique, l'acide glutamique, l'acide méthanesulfonique ou l'acide éthanesulfonique. Préférentiellement, on ajoute de l'acide ascorbique ou de l'acide lactique à la chitine. On libère ainsi les fonctions amine et on les salifie.

Le chitosane selon l'invention également est utilisé sous forme basique ayant un pH compris entre 7 et 12. On peut ainsi utiliser le chitosane succinamide, le chitosane acétamide, le chitosane tartramide et d'une manière générale les alkyl carboxamides de chitosane où le radical alkyle contient de 2 à 6 atomes de carbone.

Les compositions selon l'invention contiennent donc soit un chitosane sous forme acide soit un chitosane sous forme basique tel que défini dans la revendication 1 soit l'association d'un chitosane acide et d'un chitosane basique suivant leurs destinations et l'effet que l'on souhaite obtenir.

Dans un milieu acide tel que l'estomac, un chitosane acide qui est chargé positivement prend la forme d'un gel et fixe les acides gras chargés négativement. Il fixe également certaines protéines, les substances thérapeutiques ou les métaux.
En outre, un chitosane « acide » pourra aussi libérer certains composés qu'il aura préalablement chélatés en milieu acide. Ce relargage sera d'autant plus efficace que le pH gastrique est bas et que la fixation initiale des composés aura été accomplie en milieu peu acide.
Dans un milieu alcalin tel que le duodénum, le jéjunum et l'iléon, un chitosane basique fixera surtout certaines protéines neutres ou faiblement basiques, des polyosides ou des métaux lourds. De la même manière que le chitosane acide, le chitosane basique pourra relarger certains des principes actifs qu'il aura initialement chélatés en milieu basique. Ce relargage sera d'autant plus efficace que le pH intestinal est élevé et que la fixation initiale aura été réalisée en milieu basique.

A température ambiante et en milieu faiblement basique, le chitosane basique fixe les polysaccharides ramifiés complexes ou les protéines fortement glycosylées des champignons pour les libérer dans le duodénum, le jéjunum, l'iléon et le colon. La chélation des métaux lourds n'est que très peu réversible. On obtient ainsi une libération prolongée des principes actifs - y compris de ceux liés par la chitine moins affine que le chitosane - et une élimination des contaminants métalliques lourds.

L'association d'un chitosane acide et d'un chitosane basique ayant préalablement fixé les polysaccharides et les protéines glycosylées des champignons permet :
- d'assurer une élimination par chélation complète des contaminations (métaux lourds, etc.) ;
- d'éviter toute interaction entre les polysaccharides ou les protéines glycosylées provenant des champignons et les acides gras qui pourraient altérer leur absorption intestinale ou leurs propriétés. En outre, cette association évite également la perte des principes liposolubles.

Les compositions selon l'invention renferment de 30 à 70 % de chitosane acide ou alcalin en poids de la masse totale (champignons +chitosane). Elles sont administrées de 1 à 4 fois par jour par voie digestive en fonction des champignons qu'elles contiennent. Elles ne manifestent ni toxicité ni phénomène d'intolérance.

Les compositions selon l'invention peuvent contenir des champignons ou des mycélia frais ou sous forme d'extrait sec. En outre, on pourra utiliser leur fruit ou leur mycélium. De plus, les champignons utilisés pourront être des champignons de culture avec ou sans support de culture.

Les champignons seront choisis parmi l'Armillaire miel, l'Agaricus bisporus, le Boletus edulis, le Cordyceps sinensis, le Coriolus versicolor, la Flammulina velutipes, le Ganoderma lucidum, le Hericium erinaceus, le Hypsizygus marmoreus, l'Auricularia auricula-Judae, le Phellinus linteus, le Pleurotus ostreatus, le Grifola frondosa, l'Agaricus campestris, le Lentinus edodes, la Tremella fuciformis et la Volvaria volvacea.

Les compositions selon l'invention peuvent être en outre mélangées ou diluées avec ou dans des adjuvants diététiques comme des farines, des sucres, des polyols, des saccharides, des matériaux de charge et/ou avec des édulcorants, avec des agents liants, avec des agents aromatisants, avec des agents de sapidité.

Comme matériau de charge, on peut citer des celluloses, des celluloses modifiées, des argiles, des sels minéraux, des protéines non digestibles.

Comme agent liant, on citera des celluloses alcoylées, des carboxyméthylcelluloses réticulées ou non, des carboxyméthylamidons ou des polymères de vinyl pyrrolidone pontés.

Comme agent de sapidité, on citera des sucres, le miel, les noix, les noisettes ou tout autre produit naturel.
Les sucres qui peuvent être incorporés aux compositions selon l'invention sont des sucres digestibles comme le saccharose, le fructose, le maltose ou le lactose ou bien des sucres non digestibles comme le glucose et l'arabinose.

Les compositions selon l'invention sont présentées sous l'une des formes convenant pour l'administration par voie orale et notamment sous forme de gélules, de poudres, de capsules molles ou de granulés, mais également sous forme de galettes, de biscuits ou de tout autre support alimentaire.
Aux fins alimentaires, les compositions selon l'invention pourront également être incorporées dans des sauces, des pâtes, des pains et dans la charcuterie.
Les excipients ou diluants appropriés pour ces voies peuvent être des produits minéraux inertes, comme par exemple le carbonate de calcium, le phosphate tricalcique, le phosphate de magnésium, l'alumine, la silice colloïdale, le kaolin, les argiles, le silicate d'aluminium, le silicate de calcium ou l'oxyde de fer ou l'eau ou les liquides aqueux pour la voie orale.

Les compositions selon l'invention sont particulièrement destinées à une utilisation thérapeutique, notamment dans les maladies suivantes :
- l'obésité ;
- l'hypercholestérolémie ;
- le diabète ;
- les troubles de la mémoire ;
- le cancer;
- l'asthme.

Les compositions selon l'invention peuvent en outre être utilisées contre le vieillissement et pour pallier la dénutrition.

Les exemples suivants illustrent l'invention. Ils ne la limitent en aucune façon :

### EXEMPLE 1

### Galettes à base de Coriolus versicolor

- chitosane succinamide (pH compris entre 7 et 12) 100 g
- Coriolus versicolor 200 g
   (extrait sec entier ou mycélium sur seigle ou froment)
- sel de chitosane (pH inférieur à 6) 100 g
   + 1% d'acide ascorbique
pour 40 galettes au poids moyen de 10 g contenant chacune environ en moyenne 1,25 g de coriolus versicolor.

Les galettes selon l'invention sont préparées de la manière suivante :
- Le chitosane succinamide et le Coriolus versicolor sont mélangés à température ambiante en ajoutant de l'eau jusqu'à l'obtention d'un mélange homogène. On laisse reposer le mélange obtenu pendant une heure puis on additionne, à celui-ci, le sel de chitosane en présence d'eau et à température ambiante jusqu'à l'obtention d'un mélange homogène. Le mélange ainsi obtenu est réparti en 40 galettes que l'on passe dans un four à feu doux jusqu'à cuisson.

Les principes actifs du Coriolus versicolor étant hydrosolubles, la fixation préalable des graisses au niveau gastrique n'est pas réalisée. Toutefois, les interactions possibles entre les acides gras et les polyosides ou protéines fortement glycosylées sont évitées.

### EXEMPLE 11

### Galettes à base de grifola frondosa

- chitosane succinamide (pH compris entre 7 et 12) 100 g
- Grifola frondosa 200 g
   (extrait sec entier ou mycélium sur seigle ou froment)
- sel de chitosane (pH inférieur à 6) 100 g
   + 1% d'acide ascorbique
   pour 40 galettes au poids moyen de 10 g contenant chacune environ en moyenne 1,25 g de Grifola frondosa.

Grifola frondosa, ou polypore en touffe ou maïtaké, est un champignon comestible considéré comme produit alimentaire d'origine végétale. Il n'appartient pas à la liste des médicaments à base de plantes. L'ajout de glucosamines polymérisées comme le chitosan apporte tous les avantages envisagés : libération des principes actifs dans l'intestin grêle et « piégeage » (trapping) des métaux lourds éventuellement présents.

La préparation des galettes à base de Grifola frondosa est réalisée de manière identique à celles à base de Coriolus versicolor.

Les principes actifs du Grifola frondosa sont soit hydrosolubles, soit liposolubles. Il est particulièrement intéressant de chélater les graisses dans l'estomac et dans l'intestin, lors de la libération des principes actifs afin de bénéficier de toute la palette des actions thérapeutiques. En outre toutes les interactions entre les acides gras et les polyosides ou les protéines fortement glycolysées sont évitées.

Les préparations des exemples I et II permettent donc d'obtenir :
- une gélification du chitosane acide dans l'estomac avec fixation des graisses et des métaux lourds ;
- une gélification du chitosane basique avec libération des principes actifs du coriolus versicolor ou du grifola frondosa dans l'intestin, sans aucune dispersion. Les métaux lourds résiduels sont fixés à ce moment-là.

### EXEMPLE III

### Réalisation de biscuits à base de Grifola frondosa

Les constituants dépendent du type de biscuit: Joconde ou génoise. Ils correspondent à des produits classiquement utilisés en biscuiterie.

| **Joconde (Kcal/100 g = 350 ou 1 460 KJ)** | |
|---|---|
| **Ingrédients** | **Kcal** |
| Oeuf entier | 147 |
| Sucre | 394 |
| Farine | 341 |
| Champignon (Grifola frondosa) | 25 |
| Glucosamines polymérisées | 0 |
| Emulsifiant E472b (2.01 %) | 288 |
| Fibre d'avoine | 40 |
| Poudre levante E450ai-E500ii (0.67 %) | 29 |
| Sorbate de potassium E202 (0.23 %) | 490 |
| Bicarbonate de sodium (traces < 0.01 %) | 0 |

### EXEMPLE IV

### Réalisation de biscuits à base de Grifola frondosa

| **Génoise (Kcal/100 g = 280 ou 1 170 KJ)** | |
|---|---|
| **Ingrédients** | **Kcal** |
| Farine | 341 |
| Sucre | 394 |
| Eau | 0 |
| Oeuf | 147 |
| Champignon (Grifola frondosa) | 25 |
| Glucosamines polymérisées | 0 |
| Dinalplus* | 363 |
| Emulsifiant E472b (0.85 %) | 288 |
| Poudre levante E450ai-E500ii (0.85 %) | 29 |
| Propionate de calcium E282 (0.22 %) | 0 |
| Sel (0.01 %) | 0 |
| Bicarbonate de sodium (traces < 0.01 %) | 0 |

| | |
|---|---|
| * Dinalplus = protéines lactiques, protéines végétales (farine de pois, farine de riz, fécule de pomme de terre) | |

### Principe de fabrication

Le maïtaké est mélangé à du chitosan en milieu alcalin à une température respectant le principe actif.
Du chitosan poudre (pH neutre ou acide) est ajouté après refroidissement pour constituer le principe actif.
Le principe actif est ensuite associé à une base biscuit standard déjà commercialisée par le biscuitier. Les modalités de cuisson respectent le principe actif. Le principe actif n'altère pas la conservation du produit qui a été testée.

## Revendications

1. Compositions pharmaceutiques et/ou diététiques à base de champignons comestibles **caractérisées en ce qu'**elles contiennent un ou plusieurs champignons présentant des propriétés thérapeutiques et de 30% à 70% en poids de la masse totale soit de chitosane acide et de chitosane basique, soit de chitosane acide isolément ou de chitosane basique sous forme d'alkylcarboxamide ayant de 2 à 6 atomes de carbone en tant qu'agent de chélation d'agents contaminants.

2. Compositions selon la revendication 1, **caractérisées en ce que** le chitosane acide est un chitosane acide résultant du traitement par un acide organique de la chitine.

3. Compositions selon la revendication 1 dans laquelle le chitosan basique est un alkyl carboxamide ayant de 2 à 6 atomes de carbone.

4. Compositions selon la revendication 1 et la revendication 3 en ce que le chitosane basique est le chitosane succinamide, le chitosane acétamide, le chitosane tartramide.

5. Compositions selon la revendication 1, **caractérisées en ce que** le chitosane est un mélange de chitosane acide et de chitosane basique.

6. Compositions selon l'une des revendications 2 ou 4 cactérisées en ce que le chitosane sous forme acide, présente un pH inférieur à 6.

7. Compositions selon l'une quelconque des revendications 2, 3 et 4 **caractérisées en ce que** le chitosane sous forme acide est obtenu par action sur la chitine d'un acide organique choisi parmi l'acide acétique, l'acide lactique, l'acide succinique, l'acide tartrique, l'acide ascorbique, l'acide citrique, l'acide glutamique, l'acide méthanesulfonique ou l'acide éthanesulfonique.

8. Compositions selon l'une quelconque des revendications 2, 5 et 6 **caractérisées en ce que** le chitosane acide est obtenu par action de l'acide ascorbique ou de l'acide lactique à la chitine.

9. Compositions selon l'une des revendications 3 ou 4, **caractérisées en ce que** le chitosane basique présente un pH compris entre 7 et 12.

10. Compositions selon l'une quelconque des revendications 1 à 9, **caractérisées en ce que** les champignons ou mycélia sont utilisés sous forme fraîche ou sous forme d'extrait sec.

11. Compositions selon l'une quelconque des revendications 1 à 10, **caractérisées en ce que** les champignons sont choisis parmi : l'armillaire miel, l'agaricus bisporus, le boletus edulis, le cordyceps sinensis, le coriolus versicolor, la flammulina velutipes, le ganoderma lucidum, le hericium erinaceus, le hypsizygus marmoreus, l'auricularia auricula-Judae, le phellinus linteus, le pleurotus ostreatus, le grifola frondosa, l'agaricus campestris, le lentinus edodes, la tremella fuciformis, la volvaria volvacea.

12. Utilisation des compositions selon l'une quelconque des revendications 1 à 11, en vue de la réalisation d'une préparation diététique et/ou pharmaceutique appropriée pour combattre l'obésité, l'hypercholestérolémie, le diabète, le cancer, les troubles de la mémoire, l'asthme.

## Patentansprüche

1. Pharmazeutische und/oder dietätische Zusammensetzungen, auf der Basis von essbaren Pilzen,
**dadurch gekennzeichnet**
**dass** sie ein oder mehrere Pilzen mit therapeutischen Eigenschaften und von 30 bis 70 % bei Gewicht der ganze Masse, saures Chitosan und basisches Chitosan, oder saueres Chitosan allein oder basische Chitosan in der Form eines Alkylcarboxamids, mit von 2 bis 6 Kohlenstoffatomen, als Komplexbildner für schädigen Wirkstoffen enthalten.

2. Zusammensetzungen nach Anspruch 1,
**dadurch gekennzeichnet**
**dass** das saueres Chitosan ein Chitosan das durch die Einwirkung einer organischen Säure erzeugt wird, ist.

3. Zusammensetzungen nach Anspruch 1,
worin das basisches Chitosan eines Alkylcarboxamid mit von 2 bis 6 Kohlenstoffatomen, ist.

4. Zusammensetzungen nach Anspruch 1 und Anspruch 3,
worin das basisches Chitosan Bernsternsaure amid, Chitosan acetamide oder Chitosan Weinsäure amid ist.

5. Zusammensetzungen nach Anspruch 1,
**dadurch gekennzeichnet**
**dass** das Chitosan unter saure Form, einen pH Wert unter 6 zeigt.

6. Zusammensetzungen nach irgendwelchen Anspruch 2, 3 und 4,
**dadurch gekennzeichnet**
**dass** das Chitosan unter säure Form, durch die Einwirkung von einer organischen Säure der zwischen Essigsäure, Milchsäure; Bernsteinsäure, Weinsäure, Ascorbinsäure, Zitronensäure, Glutamsäure, Methansulfonsäure und Ethansulfonsäure gewählt ist, erzeugt wird.

7. Zusammensetzungen, nach einer der Ausprüche 2 oder 4,
**dadurch gekennzeichnet**
**dass** das Chitosan unter der säure Form einen pH Wert niedriger als 6 zeigt..

8. Zusammensetzungen nach irgendwelchen der Anspruch 2, 5 und 6,
**dadurch gekennzeichnet**
**dass** das säure Chitosan durch die Einwirkung Ascorbinsäure oder Milchsäure auf das Chitin erzeugt wird.

9. Zusammensetzungen nach einen der Anspruch 3 oder 4,
**dadurch gekennzeichnet**
**dass** das basisches Chitosan einen pH Wert zwischen 7 und 12 zeigt.

10. Zusammensetzungen nach irgendwelchen Anspruch 1 bis 9,
**dadurch gekennzeichnet**
**dass** die Pilze oder die Mycelia davon, unter der frischen Form oder unter der Form eines trocknenen Extrakt, Gebrauch finden.

11. Zusammensetzungen nach irgendwelchen Anspruch 1 bis 9,
**dadurch gekennzeichnet**
**dass** die Pilze aus der Gruppe von Armillaria honig, Agaricus bisporus, Boletus edulis, Cordyceps sinensis, Coriolus versicolor, Flammulina velutipus, Ganoderma lucidum, Hericium erinaceus, Hypsizygus marmoreus, Auricularia auricula-Judae, Phellinus linteus, Pleurotus ostreatus, Grifola frondosa, Agaricus campestris, Lentinus edodes, Tremella fuciformis, und Volvaria volvacea gewählt werden.

12. Verwendung der Zusammensetzungen nach irgendwelchen Ansprüche 1 bis 11,
im Hinblick auf dem Erzeugnis eines dietätischen und/oder pharmazeutischen Preparat für die Beseitigung des Obergewichts, der Hypercholesterolämia, des Diabetes, des Krebs, der Gehirnstörungen und der Asthma.

## Claims

1. Pharmaceutical and/or dietetary compositions based on edible mushrooms wherein they contain one or several mushrooms endowed with therapeutical properties and from 30 to 70% by weight of the whole mass, either of acidic chitosan and basic chitosan, either acidic chitosan alone or basic chitosan in the form of an alkylcarboxamide having from 2 to 6 carbon atoms, as chelating mean for contaminating agents.

2. Compositions in accordance with claim 1,
wherein the acidic chitosan is a chitosan resulting from the treatment of chitin by an organic acid.

3. Compositions in accordance with claim 1,
wherein the basic chitosan is an alkylcarboxamide having from 2 to 6 carbon atom.

4. Compositions in accordance with claim 1 and claim 3,
wherein the basic chitosan is chitosan succinamide, chitosan acetamide or chitosan tartramide.

5. Compositions according to claim,
wherein the chitosan is a mixture of acid chitosan and basic chitosan.

6. Compositions according to one of claim 2 or 4,
wherein the chitosan in acidic form, shows a pH value lower than 6.

7. Compositions according to anyone of claims 2, 3 and 4,
wherein the chitosan in the acidic form is obtained by action of an organic acid selected among acetic acid, lactic acid, succinic acid, tartaric acid, ascorbic acid, citric acid, glutamic acid, methane sulphonic acid or ethane sulphonic acid, on chitin.

8. Compositions according to anyone of claims 2, 5 and 6,
wherein the acidic chitosan is obtained by action of ascorbic acid or lactic acid on chitin.

9. Compositions according to anyone of claims 3 or 4,
wherein the basic chitosan shows a pH value comprised between 7 and 12.

10. Compositions according to anyone of claims 1 to 9,
wherein the mushrooms or mycelia are used in the fresh form or in the form of a dry extract.

11. Compositions according to anyone of claims 1 to 10,
wherein the mushrooms are selected among Armillaria honey, Agaricus bisporus, Boletus edulis, Cordyceps sinensis, Coriolus versicolor, Flammulina velutipus, Ganoderma lucidum, Hericium erinaceus, Hypsizygus marmoreus, Auricularia auricula-judae, Phellinus linteus, Pleurotus ostreatus, Grifola frondosa, Agaricus campestris, Lentinus edodes, Tremella fuciformis, and Volvaria volvacea.

12. Use of the compositions according to anyone of claims 1 to 11, with the purpose of achieving a dietetic and/or pharmaceutical composition suitable for cainter acting, obesity, hypercholesterolemia, diabetes, cancer, memory disturbances or asthma.
